# EUROPEAN PATENT APPLICATION

(11) **EP 2 813 212 A1**
(43) Date of publication of application: **17.12.2014**
(21) Application number: 13002964.8
(22) Date of filing: 10.06.2013
(51) Int. Cl.: A61K 9/00, A61K 9/70, A61K 31/519

(54) **Drug formulation using API in nanofibers**

(71) Applicant: Zentiva, a.s., 102 37 Praha 10 (CZ)
(72) Inventor: Stranska, Denisa, 460 06 Liberec-Rochlice (CZ); Erlebachova, Ivana, 463 12 Liberec-Vesec (CZ); Kral, Vladimir, 120 00 Praha 2 (CZ); Sebek, Pavel, 161 00 Praha 6 (CZ); Beranek, Josef, 160 00 Praha 6 (CZ); Dumicic, Aleksandra, 10 000 Zagreb (HR); Sedmak, Gregor, 1291 Skofljica (SI); Chvojka, Tomas, 290 01 Podebrady (CZ)
(74) Representative: Jirotkova, Ivana

(57) **Abstract**

A process of manufacturing drug products using electrospinning technology,

## Description

### Technical Field

The present invention relates to a new process of manufacturing of drug products using electrospinning technology. The approach allows to dramatically increase bioavalilability of low water soluble API as a result of accommodation of API in form of stable amorphous (preferable), or crystalline nanoparticles in polymeric nanofibers. Amorphous dispersions can be made stable and can be processed into capsules or tablets.

Poorly water-soluble drugs continue to present challenges for oral and parenteral delivery. After oral administration, the degree of absorption may be highly variable and unreliable, thus, requiring suitable formulations to improve bioavailability.

For such insoluble compounds, a successful formulation is, thereof, absolutely essential to develop an efficacious drug product.

There are several attributes associated with nanofiber membranes that allow to increase the dissolution rate, to increase solubility and finally to enhance bioavailability of poorly water soluble drugs.
- the dissolution rate of drugs can be increased by increasing the surface area exposed and available for efficient dissolution (Noyes-Whitney equation).
- amorphous form of the drug usually dominantly presented within nanofibers dissolves faster than the relevant crystalline form
- crystallization of supersaturated solution of a poorly water-soluble drug after dissolution in body fluids can be hindered by polymer and/or surfactant content of the fibers
- increase the dose or solubility via crystal modification, amorphization, complexation, micronization
- the total amount of a poorly soluble micronized drug can be reduced by decreasing it to nanoscale region (Ostwald-Freundlich equation)

There is a great challenge for the formulation of poorly soluble and poorly bioavailable drugs.

The challenge is typical of what many companies come across when confronted with poorly water soluble API's that fall within BCS Class Il and exhibit low solubility and high permeability. It is estimated that around 70% of NCE's entering discovery and development programmes are within this class.

For poorly soluble compounds, a good bioavailability is typically needed to assess the therapeutic index and the suitability of the compound for technical development. In industry, the selection of the delivery technology is not only driven by technical targets, but also by constraints, such as production costs, time required for development and the intellectual property situation.

Current approach how to address these challenges is following: developments in parenteral and oral delivery technologies and products for poorly water-soluble compounds, such as nano-suspensions, solid dispersions and liposomes. In addition, the use of biorelevant dissolution media to assess dissolution and solubility properties is described. Suggestions are also included to systematically address development hurdles typical of poorly water-soluble compounds intended for parenteral or oral administration.

The technologies currently screened include:
- Suspension / Nanosuspension (Oral)
- Co-solvent Systems (Oral, Parenteral)
- cyclodextrins (Oral, Parenteral, Topical)
- Lipid formulation: Oily liquids (Oral, Parenteral, Topical)
- Emulsions (Oral, Parenteral, Topical)
- Microemulsions (Oral, Topical)
- Mixed micelles (Parenteral)
- amorphous dispersion formulation options
- microfluidised formulations.

### Background Art

In order to enhance the delivery of drugs with limited absorption due to poor solubility/dissolution, approaches are being developed to improve the dissolution rates and solubility of drug molecules. These approaches include identification of water-soluble salts of parent drugs, preparation of stable amorphous drug formulations, inclusion of solubility enhancing agents in the dosage form, and particle size reduction. Technologies to reduce drug particle size to sub-micrometer range are being applied to product development more frequently,

Electrospinning is being considered as one of the technologies which can produce nanosized drugs incorporated in polymeric nanofibers. In vitro and in vivo studies have demonstrated that the release rates of drugs from these nanofiber formulations are enhanced compared to those from original drug substance. Thus, one option how to improve bioavailability is recently developed technology based on application of nanofibers for drug formulation.

### Nano fibers

Nanofibers exhibit special properties mainly due to extremely high surface area to weight ratio compared to conventional fibers. These Special properties make them suitable for a wide range of applications from medical to consumer products and industrial to high-tech applications for aerospace, capacitors, transistors, drug delivery systems, battery separators, energy storage, fuel cells, and information technology. The second phase details the principles of Drug Delivery System (DDS), which includes the method of drug loading and different release mechanism, further it deals with different applications of nanofiber as drug delivery vehicle.

Three distinct techniques have proven successful in routinely creating nanofibrous tissue structures: self assembly, phase separation, and electrospinning. Electrospinning as a polymer-processing technology has been known for more than 70 years and is the most simple and efficient.

Electrospinning is a very simple and versatile process by which polymer nanofibers with diameters ranging from a few nanometers to several micrometers can be produced using an electrostatically driven jet of polymer solution or polymer melt. Significant progress has been made in this process throughout the past few years and electrospinning has advanced its applications in many fields, including pharmaceutics. Electrospun nanofibers show great promise for developing many types of novel drug delivery systems (DDS) due to their special characteristics and the simple but useful and effective top-down fabricating process. The current state of electrospun nanofiber-based DDS is focused on drug-loaded nanofiber preparation from pharmaceutical and biodegradable polymers and different types of DDS. However, there are more opportunities to be exploited from the electrospinning process and the corresponding drug-loaded nanofibers for drug delivery.

Polymer nanofibers have a diameter in the order of a few nanometers to over 1 µm (more typically 50∼500 nm) and possess unique characteristics, such as: extraordinary high surface area per unit mass (for instance, nanofibers with ∼100 nm diameter have a specific surface of ∼1000m²/g), coupled with remarkably high porosity, excellent structural mechanical properties, high axial strength combined with extreme flexibility, low basis weight, and cost effectiveness *are* among others.

Another interesting aspect of using nanofibers is that it is feasible to modify not only their morphology and their (internal bulk) content but also the surface structure to carry various functionalities. Nanofibers can be easily post-synthetically functionalized (for example by chemical or physical vapour deposition). Furthermore, it is even feasible to control secondary structures of nanofibers in order to prepare nanofibers with core/sheath structures, nanofibers with hollow interiors and nanofibers with porous structures.

Economically, the electrospinning nano-manufacturing process is relatively low cost compared to that of most bottom-up nanofiber-fabricating methods. The resulting nanofibers are often uniform, continuous and do not require expensive purification protocols. The nanofibers are relatively easy to be scaled up for productivity due to the top-down process and the designing of multiple jets for synchronous electrospinning. Additionally, the nanofibers have one dimension at the microscopic scale but another dimension macroscopically. This unique characteristic endows nanofiber mats with both the merits possessed by functional materials on the nanometer scale, and these have advantages *over* conventional solid membrane such as easy processing.

These outstanding properties make polymer nanofibers as good candidates for many applications. For example nanofibers mats are now being considered for composite materials reinforcement, sensors, filtration, catalysis, protective clothing, biomedical applications (including wound dressing and scaffolds for tissue engineering, implants, membranes and drug delivery systems), space applications such as solar sails, and micro- and nanooptoelectronics. Thus the properties of nanofibers make them useful for systems for developing nanofibers-based DDS.

### Current state of electrospun nanofiber based DDS

Research about electrospun nanofibers as drug delivery systems is in the early stage of exploration. Many current researches focus on the preparation and characterization of polymer nanofibers. To date, it is generally believed that nearly one hundred different polymers, mostly dissolved in solvents yet some heated into melts have been successfully spun into ultrafine fibers. How to transit nanofibers into *DDS* is creating much attention,

The first report about electrospinning fibers were explored as drug delivery vehicles using tetracycline hydrochloride as a model drug. The mats were made either from poly (lactic acid) (PLA), poly (ethylene-co-vinyl acetate) (PEVA), or from a 50:50 blend of the two from chloroform solutions. Release profiles showed promising results when they were compared to a commercially available DDS-Actisite^{®} (Alza Corpora-tion, Palo Alto, CA), as well as to the corresponding cast films. An early patent WO0154667 registered by Ignatious and Baldoni described electrospun polymer nanofibers for pharmaceutical compositions, which can be designed to provide rapid, immediate, delayed, or modified dissolution, such as sustained and/or pulsatile release characteristics.

Later studies on the preparation of nanofibers from polymers with different drug-loaded capabilities and the corresponding DDS were reported, such as transdermal, fast dissolving and implantable DDS. Most of the early work focused on the sustained release profiles and all types of active pharmaceutical ingredients were used as model` drugs, such as small molecular drug, herbs, proteins, poorly water-soluble and water-soluble drugs, DNA, genes and vaccines. The polymers include biodegradable hydrophilic polymers, hydrophobic polymers and amphiphilic polymers.

There are report how to accommodate therapeutic peptides, proteins and heparins by this technology for DDS, but our focus has been on-modern formulation application of small molecules.

Electrospun nanofibers are often used to load insoluble drugs for enhancing their dissolution properties due to their high surface area per unit mass. Tungprapa et al, (Polymer, 48, 5030-5041) prepared ultra-fine fiber mats of cellulose acetate (CA) for four different types of model drugs, i.e., naproxen (NAP), indomethacin (IND), ibuprofen (IBU), and sulindac (SUL), from 16% w/v CA solutions in 2:1 v/v acetone/N,N-dimethylacetamide (DMAc) by electro-spinning. The amount of the drugs in the solutions was fixed at 20 wt% based on the weight of CA powder. No drug aggregates were observed on the surfaces of the fibers. The maximum release of the drugs from loaded fiber mats were ranked as follows: NAP>IBU>IND> SUL and this did not correspond to their solubility properties. Taepaiboon et a/. (Nanotechnology, 17, 2317-2329*)* reported that the molecular weight of the model drugs played a major role on both the rate and the total amount of drugs released from the prepared drug-loaded electrospun PVA nanofibers. The rate and the total amount of the drugs released decreasing with increasing molecular weight of the encapsulated drugs. Taepaiboon *et al.* also reported that mats of PVA nanofibres were successfully prepared by the electrospinning process and were developed as carriers of drugs for a transdermal drug delivery system. Besides the water insoluble drugs naproxen (NAP), and indomethacin (IND), freely water soluble sodium salicylate, was also spun into the PVA fibers. Xu et al. (Macromol, Rapid Commun. 27, 1637-1642) proposed a novel process, i.e., 'emulsion electrospinning' to prepare core-sheath fibers to incorporate a water soluble drug into a hydrophobic or an amphiphilic polymer fiber. Maretschek et al. (J. Control. Release, 127, 180-187) recently reported the electrospin-ning of emulsions composed of an organic poly (L-lac-tide) solution and an aqueous protein solution, which yielded protein containing nanofiber nonwovens having a mean fiber diameter of approximately 350 nm. This provided the opportunity to tailor the release profile of macromolecular active ingredients. All the above reports demonstrated that electrospun drug-loaded nanofibers were able to provide sustained release profiles for different types of active pharmaceutical ingredients.

Studies previously reported the influence of a high electrical potential on the chemical integrity of the drugs, the comparatively controlled release characteristics of nanofibers and the release-controlled mechanisms. Tungprapa *et al.* and Taepaiboon *et al.* confirmed that the electrospinning process did not affect the chemical integrity of the drugs by 1H-nuclear magnetic resonance. Taepaiboon et al, proved that the drug-loaded electrospun PVA mats exhibited better release characteristics of four model drugs than drug-loaded as-cast films and Tungprapa *et al.* showed that the release of drugs from the CA drug- loaded films was mainly due to the gradual dissolution of aggregates on the film surfaces, whilst the diffusion of the drugs incorporated within the films occurred to a lesser extent. On the contrary, since no presence of the drug aggregates was found on the surface of the drug-loaded CA fibers, the release of the drugs from the drug-loaded fiber mats was mainly by the diffusion of the drugs from the fibers, as the fiber mats could swell appreciably in the testing medium. Moreover the fibrous morphology of the drug-loaded fiber mats after the drug release assay at 24h was still intact. Verreck et al. (Pharm Res, 20, 810-817) confirmed that the application of electrostatic spinning to pharmaceutical applications resulted in dosage forms with better useful and controllable dissolution properties than the simple physical mixture, solvent cast or melt extruded samples.

Although many types of DDS have been prepared from electrospun drug-loaded nanofibers, no related clinical experiments have been reported and only few in vivo drug delivery researches have been undertaken, which were mainly associated with the cancer research. Ranganath^{A} *et al.* reported the paclitaxel-loaded biodegradable implants in the form of microfiber discs and sheets developed using electrospinning were used to treat malignant glioma in vitro and in vivo. The fibrous matrices not only provided greater surface area to volume ratio for effective drug release rates but also provided needed implantability into the tumor resected cavity of a post-surgical glioma.

The advantages of employing electrospinning technology to prepare DDS *are* not as yet fully exploited. Nanotechnology is now having an impact in biotechnology, pharmaceutical and medical diagnostics sciences. Nanodrugs are at the forefront of bioengineering for diseases and represent the next generation of medical therapies that will impact worldwide markets and especially the healthcare industry. Furthermore electrospinning as noted before has gained more attention due in part to a surging interest in nanotechnology, as ultrafine fibers or fibrous structures of various polymers with small diameters. On the other hand, electro-spinning should exert more influence on new DDS development through providing novel strategies for conceiving and fabricating them.

### Electrospinning Technology challenges

Although some reports in the literature have demonstrated that electrospinning is useful for preparing new DDS there are still some challenges associated with the preparation of electrospun nanofiber-based DDS.

Electrospinning is a simple micro-processing technique to make ultrafine or nanometer range fibers generally from high molecular weight polymer solutions or melts. The largest challenge lies firstly in understanding the electrospinning process as a fluid dynamics system. In order to control the properties, geometry, and mass production of the nanofibers, it is necessary to understand quantitatively how electrospinning transforms the fluid solution through a millimeter diameter capillary tube into solid fibers which are four to five orders smaller in diameter. Secondly, the efficiency of electrospinning is still a bottleneck. Studies on multiple nozzles need to be undertaken and these will form a platform for electrospinning industrialization.

To date, most of the release tests have been done in vitro. What is more, several problems must be resolved for further applications such as the drug loading, the initial burst effect, the residual organic solvent, the stability of active agents, and the combined usage of new biocompatible polymers. Drug-loading is always a problem for nano DDS. Although drug loading over 50% of the total weight was reported, the drug loading in the nanofibers still needs to be increased in many cases.

### Nanospider^{™} Electrospinning Technology

Nanospider^{™} technology is a patented, needle-free high voltage, free liquid surface electrospinning process. The technology is based upon the discovery, that it is possible to create Taylar Cones and the subsequent flow of material not only from the tip of a capillary, but also from a thin film of a polymer solution. The technology enabled Elmarco to build industrial scale production equipment with out nozzles, needles or spinnerets.

Nanospider^{™} technology allows the production of nanofibers from polymers solved in water, acids or bipolar solvents. This versatile technology is easily adapted to a variety of process parameters for the optimization of the specific properties of the produced nanofibers.

Nanospider^{™} technology provides:
- High productivity and scalability
- High fiber diameter and web uniformity
- Economical operation and easy maintenance
- Flexibility in used polymers and substrates

### simplicity

Nanospider™ technology uses simply shaped electrodes, partially submerged in a polymer solution; it is mechanically simple and has no parts easily clogged (in comparison to needle-type electrospinning).

### Productivity & Quality

The numbers of fibers per machine width is given by the distance of the Taylor cones. Nanospider^{™} free liquid surface electrospinning lets natural physics define this distance, rather than using individual needles. This allows higher fiber packing density and thus an increased productivity as well as better fiber homogeneity and more consistent web morphology,

### Individuality

Nanospider™ technology allows optimizing the results in nanofiber production through precise adjustment of many process parameters, which are decisive for final product:
- Solution parameters (conductivity, temperature, surface tension, etc.)
- Environmental parameters (temperature, humidity etc.)
- Base material parameters (cross and surface electrical resistance, etc.)
- Equipment parameters (voltage, electrode distance, etc.)

### Biopolymers

Nanospider™ technology allows to spin a lot of types of polymers primarily design for medical or pharmaceutical application. We lay emphasis on the selection of suitable solvents system therefore we try to maximize the use of water or organic acid solvents for electrospinning, Nanospider™ technology enables to produce nanofibers from polylactid acid, polycaprolactone, chitosane, gelatin, polyacrylic acid, hydroxypropylcellulose, etc.

### Advantages of Nanosplder^{™} technology

- Prepared nanofiber layer is very flexible without use of plasticizers or stabilizers and it can be dissolved fast, medium or slowly by a proper choice of polymers and area weight of nanofibers.
- Electrospinning process is operated with laboratory temperature which is very important for thermally unstable drugs,
- The drug loading capacity can be high, between 40-60% related to the mass of fibers.
- The composition of nanolayers enables to ensure suitable taste masking.
- The API's are present in the amorphous phase which is an effect of electrospinning desirable and very important for drug release.

### Dissolution study

Drug delivery with polymer nanofibers is based on the principle that dissolution rate of a drug particulate increases with increasing surface area of both the drug and the corresponding carrier if necessary. For controlled drug delivery, in addition to their large surface area to volume ratio, polymer *nanofiber* also have other additional advantages, controlled delivery systems are used to improve the therapeutic efficacy and safety of drugs by delivering them to the site of action at a rate dictated by the need of the physiological environment. A wide variety of polymeric materials have been used as delivery matrices, and the choice of the delivery vehicle polymer is determined by the requirements of the specific application. Polymeric nanafibers have recently been explored for their ability to encapsulate and deliver bioactive molecules for therapeutic applications.

### Drug loading

One method to incorporate therapeutic drugs into nanofibers involves solubilizing the drug into the polymer solution to be spun. Using this method, a loading efficiency of 90% into PDLA nanofibers was reported for the antibiotic drug Mefoxin. Covalent conjugation to polymers represents another method to modulate drug release. It has also been suggested that the high porosity of nanofibers allows for rapid diffusion of degradation byproducts. However, the burst release may also be indicative of the drug being attached only on the surface. As the drug and carrier materials can be mixed together for electrospinning of nanofibers, the likely modes of the drug in the resulting nanostructed products are:
1. Drug as particles attached to the surface of the carrier which is in the form of nanofibers,
2. Both drug and carrier are nanofiber form; hence the end product will be the two kinds of nanofibers interlaced together,
3. The blend of drug and carrier materials integrated into one kind of fibers containing both components, and
4. The carrier material is electrospun into a tubular form in which the drug particles are encapsulated.

### Mechanism of drug delivery

Nanofiber drug delivery systems may provide insight into the direct incorporation of bioactive growth factors into scaffolds. Additionally, drug delivery systems can be combined with implantable tissue engineering scaffolds to prevent infection while repair and regeneration occur. Biodegradable polymers release drug in one of two ways: erosion and diffusion. Release from biodegradable polymers in vivo is governed by a combination of both mechanisms, which depends on the relative rates of erosion and diffusion. Most biodegradable polymers used for drug delivery are degraded by hydrolysis. Other biodegradable polymers are enzymatically degradable, which is also a type of chain scission. As water molecules break chemical bonds along the polymer chain, the physical integrity of the polymer degrades and allows drug to be released. The different mechanisms were given below.

### Essence of the invention

The present invention relates to a new process of manufacturing of drug products using electrospinning technology. The approach allows to increase bioavalilability of low water soluble API as a result of accommodation of API in form of stable amorphous (preferable), or crystalline nanoparticles in polymeric nanofibers. Two different types of polymer were used for production of nanofiber drug loading membrane, polyvinylpyrrolidon and Soluplus. Amorphous dispersions can be made stable and can be processed into capsules or tablets,

### Detailed disclosure

The advantages of employing electrospinning technology to prepare DDS are not as yet fully exploited. Nanotechnology is now having an impact in biotechnology, pharmaceutical and medical diagnostics sciences. Furthermore electro-spinning as noted before has gained more attention due in part to a surging interest in nanotechnology, as ultrafine fibers or fibrous structures of various polymers with small diameters40. On the other hand, electro-spinning should exert more influence on new DDS development through providing novel strategies for conceiving and fabricating them. Still several problems must be resolved for further applications such as the drug loading, the initial burst effect, the residual organic solvent, the stability of active agents, and the combined usage of new biocompatible polymers,

The drug delivery composition of this invention is a nonwoven nanofiber web or mat containing an active ingredient or ingredients. Preferably the active ingredient is dispersed throughout a matrix comprising the nanofiber web, although the invention also provides a nanocomposite wherein the active ingredient is loaded in, or adsorbed to, a vehicle comprising the nanofiber web.

A nanofiber web or mat for the purposes of this invention is a nonwoven randomly oriented or aligned collection of nanofibers. These nanofiber webs or mats are typically in the form of a thick and tangled mass defined by an open texture or porosity. For the purposes of this disclosure the terms nanofiber membrane, nanofiber web, nanofiber mat and nanofiber mesh are used interchangeably. The nanofiber web or mat is a membrane. Macroscopically, the membrane is a network of nanofibrous structure.

Nanofibers can be formed from various inorganic, organic, or biological polymers to form the nanofiber mat. Preferably these nanofibers are formed by electrospinning, However, other techniques such as drawing, template synthesis, phase separation or self-assembly may be used to produce nanofibers. All of these techniques are described in "An Introduction to Eloctrospinning and Nanofibers", Ramakrishna et al., World Scientific, 2005, Nanofiber mats or webs can be modified by compression into pellets; by folding into homogeneous or heterogeneous layers; cutting into discs or rings; laminating onto carrier polymers, films, fabrics (woven or nonwoven), paper, or biological membranes; or chopped into short segments known as whiskers.

The nanofibers are preferably less than 3 micrometers in diameter, more preferably less than 500 nm in diameter, and most preferably less than 500 nm in diameter and greater than 2 nanometers in diameter. The thickness of the nanofiber web is less than 10 mm, more preferably less than 5 mm in thickness, and most preferably less than 1 mm in thickness. The weight of the active ingredient in the nanofiber web is less than 80 weight percent of the total weight of the active ingredient and the nanofiber web, more preferably less than 50 weight percent, and most preferably less than 20 weight percent.

Preferably, the polymers used to make the nanofibers need to be blocompatible. For the purposes of this patent, biocompatibility means the capability of coexistence with living tissues or organisms without causing harm by not being toxic, injurious, or physiologically tissues or organisms without causing harm by not being toxic, injurious, or physiologically reactive and not causing immunological rejection. The polymers used to make the nanofibers can be biodegradable or non-biodegradable and synthetic or natural. Examples of biocompatible, biodegradable synthetic polymers include but are not limited to PVP, soluplus polyesterurethane (Degrapol^{®}), poly(ε-caprolactone), polydioxanone, Poly(ethylene oxide), polyglycolide, poly(lactic acid) (PLA), poly(L-lactide-co-ε-caprolactone), poly(lactide-co-glycolide) (PLGA). One of the greatest potentials of electrospun fibers is in the field of tissue engineering.

The composition comprising ticagrelor and at least one second component used to stabilize amorphous ticagrelor typically comprises at least one filler. Water-soluble fillers are generally preferred. Suitable fillers comprising ticagrelor composition may be selected from, but not restricted to monosaccharides, oligosaccharides and sugar alcohols like glucose, fructose, saccharose, lactose monohydrate, lactose anhydrous, raffinose, isomalt, trehalose, dextrates, mannitol, erythritol, sorbitol, maltitol, xylitol and lactitol, compressible sugar, dibasic calcium phosphate dihydrate and mixtures thereof. Preferred fillers are lactose, mannitol, xylitol. The composition comprising ticagrelor and at least one second component used to stabilize amorphous ticagrelor typically comprises binder. Suitable binders may include povidone, copovidone, cellulose powder, crystalline cellulose, microcrystalline cellulose, siliconized microcrystalline cellulose, cellulose derivatives such as hydroxymethylcellulose, hydroxyethylcellulose, hydroxypropylcellulose and hydroxypropylmethylcellulose, maltose, starch, pregelatinized starch, polymethacrylates and mixtures thereof. Hydroxypropylcellulose and maltose are particularly preferred. The composition comprising ticagrelor preferably comprises 0.5 to 30 wt.-% of binder, more preferably 1 to 7 wt.-% of binder, most preferably 2 to 4 wt.-% of binder.

The composition comprising ticagrelor and at least one second component used to stabilize amorphous ticagrelor typically comprises surfactant. Suitable surfactants may be selected from anionic, cationic, ampholytic and non-ionic surfactants such as sodium lauryl sulfate, cetrimide, N-dodecyl-N,N-dimethylbetaine, polysorbates (such as Tweens^{®}), poloxamers and mixtures thereof. Preferred is sodium lauryl sulfate. The composition comprising ticagrelor preferably comprises 0.1 to 4.0 wt.-% of surfactant, most preferably 0.5 to 2.0 wt,-% of surfactant.

The composition comprising ticagrelor and at least one second component used to stabilize amorphous ticagrelor typically comprises lubricant and/or glidant. Suitable lubricants and/or glidants may be selected from magnesium stearate, hydrogenated vegetable oil, hydrogenated castor oil, talc, sodium stearyl fumarate, colloidal silicon dioxide, magnesium trisilicate and mixtures thereof. Stearic acid, magnesium stearate and sodium stearyl fumarate and colloidal silicon dioxide are particularly preferred.

The tablet and/or capsule comprising ticagrelor and at least one second component used to stabilize amorphous ticagrelor can optionally be coated by any conventional coating, Suitable coating agent may be selected from methylcellulose, hydroxypropylmethyl cellulose, hydroxypropylcellulose, acrylic polymers, ethylcellulose, cellulose acetate phthalate, polyvinyl acetate phthalate, hydroxypropyl methylcellulose phthalate, polyvinylalcohol, sodium carboxymethylcellulose, cellulose acetate, cellulose acetate phthalate, gelatin, methacrylic acid copolymer, polyethylene glycol, shellac, sucrose, titanium dioxide and carnauba wax. Preferred are polyethylene glycol, hydroxypropylmethyl cellulose and polyvinylalcohol.

### Short description of the figures

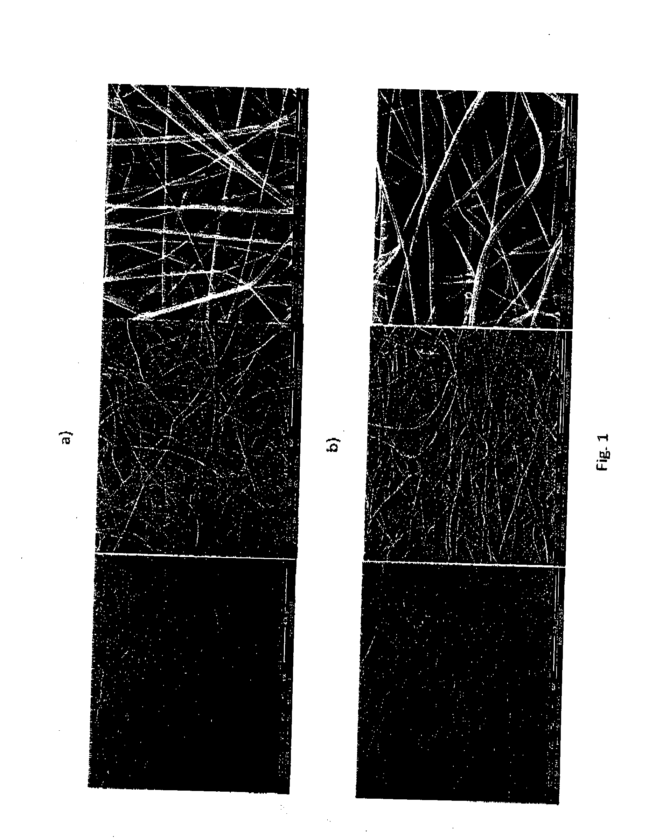

Fig. 1 depicts results of PVP electrospinning with added Ticagrelor; in Fig. 1a fiber diameter 209 ± 83.5 nm was used; in Fig. 1b fiber diameter 273 ± 96.8nm was used.

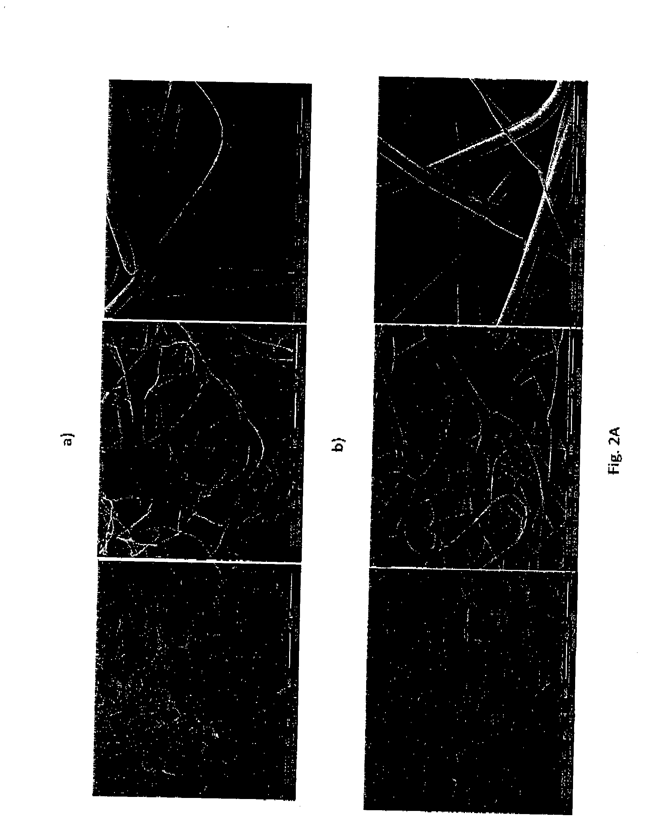

Fig. 2A depicts results of Soluplus electrospinning with added Ticagrelor; in Fig. 2Aa fiber diameter 1801 ± 894 nm was used; in Fig. 2Ab fiber diameter 1235 ± 718nm was used.

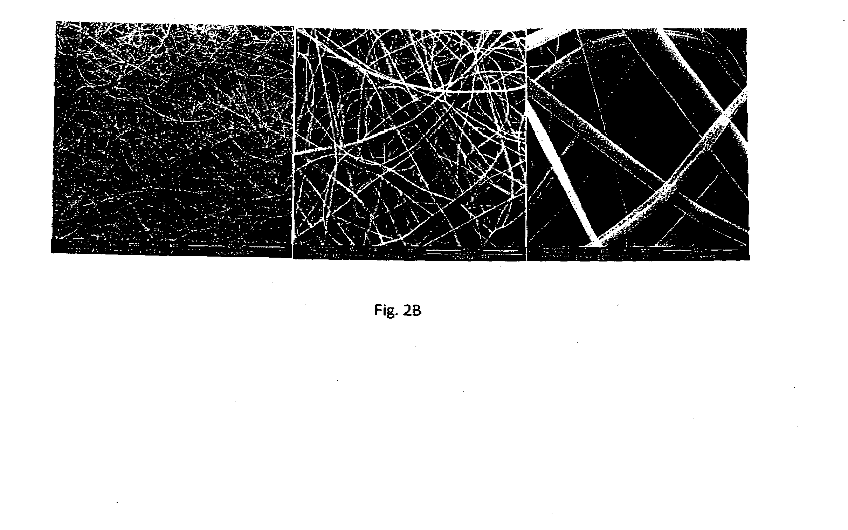

Fig. 2B depicts Soluplus electrospinning with added Telaprevir; fiber diameters 1247 ± 446 nm were used.

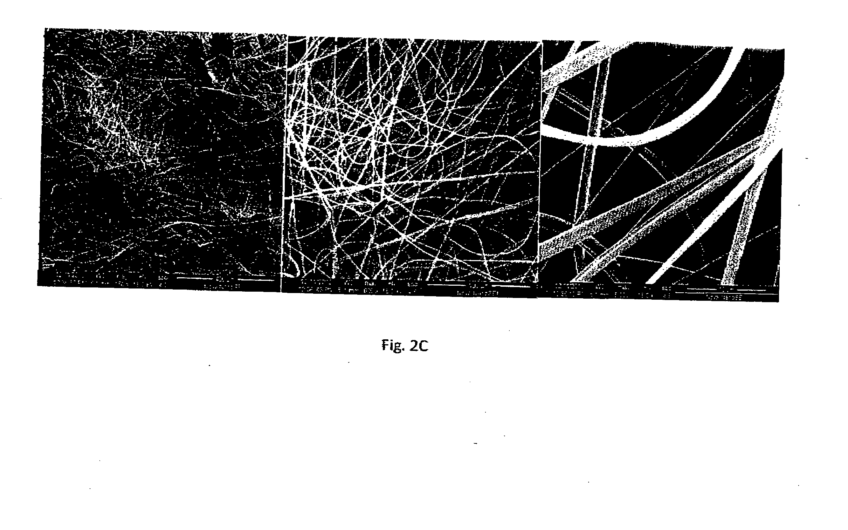

Fig. 2C depicts Soluplus electrospinning with added Aprepitant; fiber diameters 1125 ± 468 nm were used.
Fig. 3 shows results of dissolution tests.

### Feasibility study of new types of drug

All experiments were running with Nanospider™ machine, types NS_1WS500U. This is a semi-industrial equipment for nanofiber layer production with 500mm width of support textile (PP spunbond). Nanospider consists of wire to spin fibers directly from the polymer solution. This wire spinning electrode is partially wetted with the polymer solution. A grounded collector electrode is placed at the top of the spinner. Support material moves along the collector electrode which makes the production of the nanofiber layer a continuous process. Many Taylor cones are simultaneously formed on the surface of the wire spinning electrode, which makes the technology highly productive.

Two different types of polymer were used for production of nanofiber drug loading membrane, polyvinylpyrrolidon and Soluplus (polyvinyl caprolactam-polyvinyl acetate-polyethylene glycol graft co-polymer),

### Example 1 - PVP electrospinning

Polyvinylpyrrolidon (Povidone K-90) was dissolved in ethanol (98%) with continuous stirring at room temperature, concentration 5.6wt%. For better electrospinning properties 3% solution of polyethyleneoxide (PEO) was added into the PVP. Different types of drug were added with stirring into the prepared solution of polymers. After dissolution the solution was poured into to tank and process electrospinning was started. PVP is spinnable at room temperature and 23-25% relative air humidity. Set up of machine: applied high voltage 60/-10kV, distance of electrodes 170mm, ginger 0.6mm.

### Example 1A - PVP electrospinning with added Ticagrelor

0.84g PVP was dissolved in 14.16g of ethanol, 0.8g of 3% PEO was added. a) 0.0839 g of Ticagrelor was added into the solution and spun. Fiber diameter 209 ± 83.5 nm b) 0.1261g of Ticagrelor with fiber diameter 273 ± 96.8nm,

### Example 2 - Soluplus electrospinning

Soluplus (BASF) was dissolved in ethanol (98%) with continuous stirring at room temperature, concentration 32,5-33wt %. Different types of drug were added with stirring into the prepared solution of polymers. After dissolution the solution was poured into to tank and process electrospinning was started. Soluplus is spinnable at room temperature with 35-40% relative air humidity. Set up of machine: applied high voltage 33/-10kV, distance of electrodes 140mm, ginger 0,9mm.

### Example 2A - Soluplus electrospinning with added Ticagrelor

1. 6.6g of Soluplus was dissloved in 13.4g ethanol, a) 0.66g of drug was added into to polymer solution 9wt% and spun. Fiber diameter 1801±894 nm and b) 1.32g, 16.6wt% with fiber diameter 1235 ± 718nm.

### Example 2B-Soluplus electrospinning with added Telaprevir

6.5g of Soluplus was dissolved in 13.5g of ethanol and 0.4g of Telaprevir was added with stirring and spun, fiber diameters 1247 ± 446 nm

### Example 2C-Soluplus electrospinning with added Aprepitant

6.5g of Soluplus was dissolved in 13.5g of ethanol and 2.044g of Aprepitant was added with stirring and spun, fiber diameters 1125 ± 468 nm.

### Example 3 - Electrospinning - tablets comprising Ticagrelor

| | 1A | 1B | 1C | 1D |
|---|---|---|---|---|
| Ticagrelor | 90.00 | 90.00 | 90.00 | 90.00 |
| Povidone | 90.00 | 180.00 | | |
| Soluplus | | | 90.00 | 180.00 |
| Mannitol | 117.00 | | | 27.00 |
| Lactose monohydrate | | 27.00 | 117.00 | |
| Magnesium stearate | 3.00 | | 3.00 | |
| Sodium stearyl fumarate | | 3.00 | | 3.00 |
| Total (mg) | 300.00 | 300.00 | 300.00 | 300.00 |

Ticagrelor and either povidone or soluplus are dissolved in ethanol (examples 1A,2A). Solution is electrospun in order to obtain amorphous ticagrelor together with a second component in order to stabilize amorphous ticagrelor. Electrospun material is subsequently mixed with either manitol or lactose and magnesium stearate or sodium stearyl fumarate and compresed to tablets.

### Example 4 Electrospinning - capsules comprising Ticagrelor

| | 2A | 2B | 2C |
|---|---|---|---|
| Ticagrelor | 90.00 | 90.00 | 90.00 |
| Povidone | 150.00 | | |
| Soluplus | | 225.00 | 150.00 |
| Total (mg) | 240.00 | 315.00 | 240.00 |

Ticagrelor and either povidone or soluplus are dissolved in ethanol (examples 1A,2A). Solution is electrospun in order to obtain amorphous ticagrelor together with a second component in order to stabilize amorphous ticagrelor. Electrospun material is inserted into a standard hard gelatine capsule of size 0 elongated.

One type of a second component used to stabilize amorphous Ticagrelor are polymers. Suitable polymers used to stabilize amorphous ticagrelor may be selected from, but not restricted to water soluble as well as water insoluble polymers. Typical water soluble polymers suitable to stabilize amorphous ticagrelor are povidone, copovidone, hypromellose, hydroxypropylcellulose, polyvinyl caprolactam-polyvinyl acetate-polyethylene glycol graft co-polymer (Soluplus™), polyvinyl alcohol and similar. Typical water insoluble polymers suitable to stabilize amorphous ticagrelor are methylcellulose, ethylcellulose, polymethacrylates, hypromellose phthalate, hypromellose acetate succinate, cellulose acetate phthalate, carboxymethyl ethyl cellulose and crospovidone.

### Example 5- Method of incorporation of ASA into PU

The aspirin (acetylsalicylic acid, ASA) was incorporated into polyurethane in 3 different concentrations: 1%, 5%, and 10% ASA by mixing polyurethane (PU) and respective w/w concentrations in N,N-dimethylformamide (DMF) solvent. The mixed polymer solution was injected via a syringe pump and electrospun onto a grounded drum under high dc voltage under usual conditions.

### Example 6 - Method of incorporation of abiraterone acetate into PLGA

The steroid derivative abiraterone acetate was incorporated into the biodegradable polymer poly (lactide-co-glycolide) (PLGA] by electrospinning as described in Example 7. A polymer solution of 0,11% TA was mixed with 9.01 gm PLGA in 2 ml tetramethylfuran (TMF) and 15 ml DMF (36.25% polymer). The polymer-drug solution was injected via a syringe pump and electrostatically spun at 16 and 24 kV. The formed nanofibers were collected as a non-woven fabric.

### Dissolution data

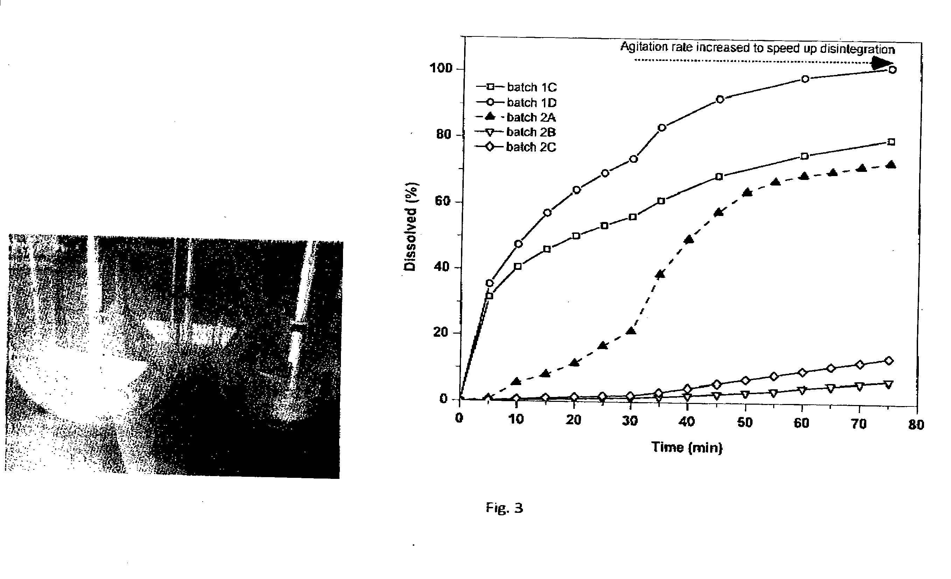

Dissolution of Ticagrelor nanofiber formulation is described in this example. As shown in the photograph in Fig. 3, nanofibers exhibit very good wettability properties leading to a sufficiently fast rate of dissolution. Selected polymer was a key factor directly determining API release rate. It was demonstrated that Povidone was a better choice than Soluplus in this particular case. Yet, it was observed that excipients externally added to a Soluplus nanofiber formulation may significantly improve capsule opening and/or tablet disintegration which directly affects API release profile.

### Drug elution

Preliminary results of drug loading of ASA into PU demonstrated a burst release of the water-soluble ASA. Demonstration of sustained release of TA from the biodegradable polymer PLGA will be performed with timed assays by ultraviolet-visible light spectroscopy. After assessing nanofiber mat uniformity, multiple round samples will be punched from the TA-PLGA nanofiber mat using a 6 mm metal punch. The 0.25 mm thin membranes will be stripped from their paper backing with jeweler's forceps and placed in closed-centrifuge vials. A 2 ml aliquot of phoshate-buffered saline (PBS) will be pipetted into the tubes with an adjustable Thermo^{®} volumetric pipette, totally immersing the samples, Triplicate samples of the drug polymer concentrations will be analyzed for timepoints 0, 24 hrs, 48 hrs, 72 hrs, 96 hrs and at weekly intervals for 6 months. At time 0, PBS will be immediately removed for analysis from the appropriate vial, leaving the nanofiber sample. Fresh PBS (2 ml) will be added back to the tube. All other timepoint samples will be incubated at 37° C. in a water-jacketed Napco incubator with periodic vortexing. At the designated sample times, the PBS will be removed and replaced with fresh 2 ml aliquots of PBS. The extractant from each timepoint will be refrigerated prior to analysis by high performance liquid chromatography (HPLC) and/or immunologic methods such as enzyme-linked immusorbent assay (ELISA). A sustained release of TA over a period of four to six months will be obtained using the procedure above.

In vitro and in vivo studies have demonstrated that the release rates of drugs from these nanofiber formulations are enhanced compared to those from original drug substance. This technology has the potential to be used for enhancing the oral delivery of poorly soluble drugs.

### Method of Delivery

For intravitreal drug delivery, a nanofiber pledget will be inserted into the vitreous cavity via a pas plana approach. For human use, a 2 mm×6 mm pledget of drug-nanofiber mat will be inserted 4 mm posterior to the limbus in phakic eyes and 3.5 mm posterior to the limbus in aphakic eyes. A triangular flap of conjunctiva is reflected in the inferior globe, exposing tenon's capsule. A similar triangular flap of tenon's capsule is created, down to bare sclera. Using a microvitreoretinal blade (MVR), a perforating sclerotomy is created with the tip of the blade directed toward the optic nerve. Next the nanofiber-drug pledget is placed directly over the sclerotomy. Using the tip of the MVR blade, the pledget is inserted into the vitreous cavity by folding the pledget at the midpoint. A single 10-0 nylon "x" suture is used to close the sclerostomy. Any vitreous wicks will be severed with Wescott scissors. Tenon's capsule and conjunctiva are teased back into place and sutured into position.

Similarly, surgical delivery of nanofiber mats may be improved by folding and/or compressing the nanofiber mats into pellets which may be injected into the vitreous cavity via the pars plana.

## Claims

1. Process for manufacturing nanofibers, wherein full formulation composition for active API BCS II and IV is generated from a solution, or suspension of API, polymeric excipients and all other formulation components for the given formulation,

2. Process according to claim 1, comprising incorporation of API into nanofibers, **characterized in that** it involves solubilization of the drug into the polymer solution prior to formation of nanofibers via electrospinning.

3. Process according to claim 2, **characterized in that** the drug is In amorphous form or in a crystalline form.

4. Process according to claim 2, wherein the drug is selected from Ticagrelor, telaprevir, Aprepitant, ASA, and abirateron acetate.

5. Process according to claim 2, **characterized in that** the drug is solubilized in a polymer selected from polyvinyl caprolactam-polyvinyl acetate-polyethylene glycol graft co-polymer, polyvinylpyrrolidone, hydroxypropylcellulose, poly(lactide-co-glycolide), poly-(lactide) (PLA), polycaprolactone, poly-(vinyl alcohol), biodegradable polyester, chitosan, poly-(propylene carbonate), poly-(lactide-glycolide), and polyurethane.

6. Process according to claim 2, **characterized in that** the polymer solution comprises a use of a solvent selected from ethanol, DMF, and TMF,

7. A drug delivery composition according to claim 1, wherein the thickness of the nanofiber web is less than 5 mm.

8. A drug delivery composition according to claim 1, wherein the nanofiber web is selected from a form consisting of a pledget, whiskers, pellet, contact lens, shield, and disc.

9. The method of drug delivery according to claim 1, wherein the nanofiber web is placed in tablet, capsules, or strips.

10. Process according to claim 1, **characterized in that** Ticagrelor and polyvinyl caprolactam-polyvinyl acetate-polyethylene glycol graft co-polymer or polyvinylpyrrolidone are dissolved in ethanol, the solution is electrospun and optionally subsequently mixed with at least one filler and at least one lubricant or surfactant.

11. Process according to claim 10, **characterized in that** the resulting material is inserted into capsules or compressed to tablets.

12. Process according to claim 10, **characterized in that** filler is selected from mannitol or lactose.

13. Process according to claim 10 **characterized in that** the surfactant is sodium stearyl fumarate.

14. Process according to claim 10 **characterized in that** the lubricant is magnesium stearate.

15. Process according to claim 1 **characterized in that** ticagrelor and polyvinyl caprolactam-polyvinyl acetate-polyethylene glycol graft co-polymer or polyvinylpyrolidone are dissolved in ethanol, the solution is electrospun and resulting material is mixed with either mannitol or lactose and magnesium stearate or sodium stearyl fumarate and encapsulated into a hard gelatine capsule or compressed to tablets.
